# EUROPEAN PATENT SPECIFICATION

(11) **EP 1 767 172 B1**
(45) Date of publication and mention of the grant of the patent: **03.02.2010**
(21) Application number: 04738339.3
(22) Date of filing: 05.07.2004
(51) Int. Cl.: A61F 9/007

(54) **AUTOMATIC CORNEA EPITHELIUM SEPARATING APPARATUS**
AUTOMATISCHES HORNHAUTEPITHEL-TRENNGERÄT
APPAREIL AUTOMATIQUE DE SEPARATION DE L'EPITHELIUM CORNEEN

(30) Priority: 26.05.2004 CN 200420023091
(43) Date of publication of application: 28.03.2007
(73) Proprietor: Wuxi Kangming Medical Device Co., Ltd, 214031 Wuxi, Jiangshu (CN)
(72) Inventor: CHU, Renyuan, 200031 Shanghai (CN); ZHANG, Baohua, 214031 Wuxi, Jiangshu (CN)
(74) Representative: Jaunez, Xavier
(86) International application number: PCT/CN2004/000741
(87) International publication number: WO 2005/115283

(56) References cited:
- WO-A-98/48748
- WO-A1-02/083042
- WO-A1-02/087451
- CN-A- 1 426 767
- CN-Y- 2 520 160
- US-A- 5 464 417
- US-A- 5 964 776
- US-A1- 2003 060 840

## Description

### Field of the invention

The present invention relates generally to a medical apparatus. More particularly, the present invention relates to a laser surgery apparatus for the use of refraction correction called "epithelial microkeratome", which is an Epi-LASIK pressure-free separation device, in particular, to a laser subepithelial keratomileusis (LASEK). The device is a set that separates corneal epithelium from Bowman's membrane with mechanical force instead of exposure to alcohol or other chemicals.

### Background Art

Refractive surgery, firstly performed in the 90's, has developed from initial PRK to LASIK and LASEK. LASIK creates a corneal stromal flap, while LASEK creates a corneal epithelial flap. The former procedure may induce more wavefront abberations and negative operational consequences such as corneal complications and glare, and the latter procedure may cause severe irritation, requiring longer operation time and higher technique, while resulting in lower success rate and slower recovery after surgery, due to alcoholic toxicity. The induction of wavefront abberation requires higher quality of excimer laser surgery. And customized ablation even enhanced the requirement of corneal flaps. In the national invention patent "A Corneal Lamella Pressureless Blade Withdrawal Device" (CN-1618414-A) filed on November 19, 2003 , by the present applicant, a rotating microkeratome was disclosed as a "pressure-free blade withdrawal device", in which the corneal flap was in a pressure-free state after cutting of cornea and withdrawal of blade, thus enabled the creation of ultra-thin corneal flaps. During practice, however, the applicant has found that the blade is still a traditional disposable stainless steel blade, which is too sharp to maintain the integrity of Bowman's membrane. Through further practice, the applicant has found that direct application of a "separation device by mechanical force" and elimination of the pressure exerting construction in the anterior surface of blade ensured direct and quick separation of corneal epithelium from Bowman's membrane. And suitable sharpness of the blade ensured integrity of corneal epithelium without incursion into the stroma. The present utility model is a device of creating epithelial flap which fulfills such requirement. It is a kind of LASEK without exposure to alcohol. It separates epithelium directly by mechanical force, and, therefore, is called Epi-LASIK, improving excimer laser surgery.

The closest state of the art is illustrated by US 2003/060840 A which discloses an automatic corneal epithelial separation device including:
a dual-motor assembly comprising two motors, a connecting key fixed below the assembly;
a separator, with an inner-round via-hole connecting up and down positioned inside, a separating piece positioned on its one side, a check arm extending out on its upper surface; and
a suction ring assembly comprising a suck ring, on the upper plane of which a suck ring stud is fixed, on the ring face of which a flat key slot is positioned, and a connecting tube connected with the suck ring integrally through a stop ring fixed at its one end.
in accordance to the preamble of claim 1.

### Summary of the Invention

The objective of the present invention is to provide a mechanical separation device, which can separate corneal epithelium from Bowman's membrane. It does not use alcohol and other chemicals, so it avoids epithelium from being loosened. It separates epithelium continuously and protects epithelial flap and stroma bed after separation.

In order to accomplish the above mentioned objective, the present invention provides a device of the above type, **characterized in that** the advancing speed of the separator is not greater than 1.5 mm/sec and the pitching-in angle between the separating piece and the cornea is less than 35°, in accordance to the characterizing portion of claim 1.

### Brief Description of the Drawings

In the following referring to the figures, the above mentioned and other objectives, features and advantages will be apparent to those skilled in this technical field from the detailed description of the present invention.
Fig. 1 is a schematic drawing of resolved construction of the present invention;
Fig. 2 is a working state of the present invention.

### Detailed Description of the Preferred Embodiments

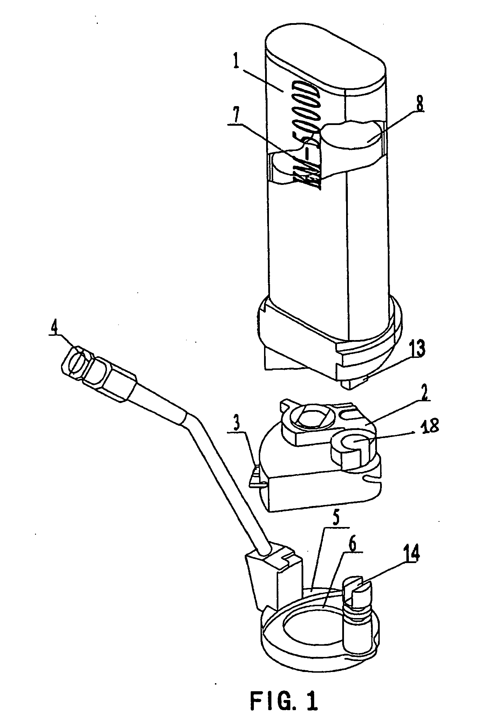

Referring to Fig. 1, the automatic corneal epithelial separation device of the present invention comprises a dual-motor assembly 1 and a separator 2. Two motors 7 and 8 are positioned inside assembly 1 and a connecting key 13 is fixed below assembly 1. The separator 2 has an inner-round via-hole connecting up and down and a separating piece 3 fixed on its one side. Moreover, the device has a suction ring assembly consisting of a suction ring 5 and a suction ring stud 12 fixed on the upper plane of the suction ring 5. A flat key slot 14 is set on the ring face of the suction ring stud 12. The device also has a connecting tube 4 with a fixed stop ring at its one end that connects with the suction ring 5 to make the device into integration.

During assembling, the connecting key 13 is inserted into the inner-round via-hole 18 of the separator 2 to make the inner-round via-hole 18 tightly fit with the suction ring stud 12 of the suction ring assembly. The flat key slot 14 and the connecting key 13 below the dual-motor assembly 1 are clutched with each other. The three parts are combined to a linkage system.

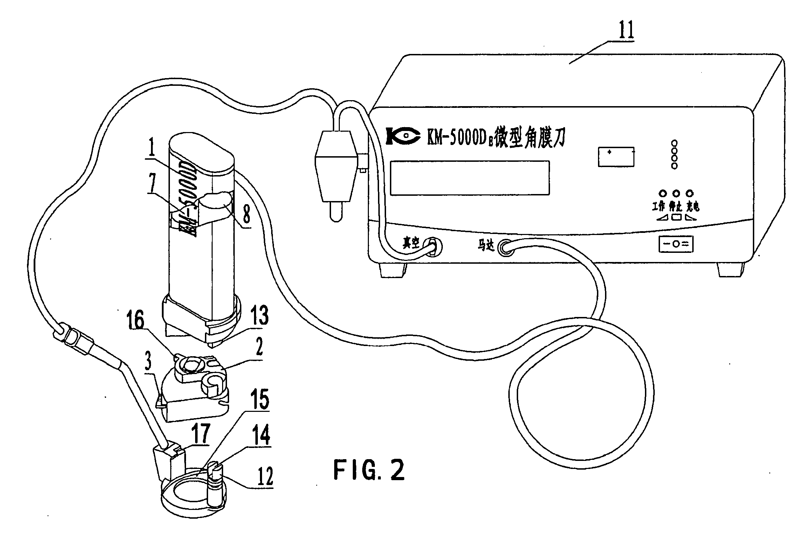

In the following referring to Fig.2, the working process of the present invention is described.

As shown in Fig.2, prior to separating corneal epithelium, the separating piece 3 is inserted into separator 2 and then combined integrally with dual-motor assembly 1. At this time, suction ring 5 has been sucked stably on the eyeball under the action of negative pressure and has overlapped stud 12 of suction ring 5. Then, separating operation is performed, during which high-speed motor 7 is started up to make separating piece 3 moving with high speed in separator 2, and slow-speed motor 8 drives dual-motor assembly 1 together with separating piece 3. Separator 2 which attaches upper plane 15 of suction ring automatically rotates counterclockwise around stud 12 on suction ring 5 until check arm 16 on separator 2 touches check hump 17 on separator 2 and the separating operation stops. Then the withdrawal operation is performed, during which slow-speed motor 8 drives dual-motor assembly 1 in counter direction, that is, clockwise moves the blade back to its original position.

During the separating procedure, the corneal epithelium does not receive positive pressure from separator 2. The operation design is a new scheme that the corneal epithelium and Bowman's membrane are automatically separated by direct mechanical force in a pressure-free state. So, the epithelial flap after surgery is perfect without any abnormalities such as tearing, and flap-related complications are fundamentally avoided.

Separator 2 in the device is a dock-styled flexible separator, that is, separating piece 3 in separator 2 is not rigidly connected, but with a clearance below spring nail with floating range less than 200 µm. The epithelium is separated while exerting flexible pressure on cornea. During cutting and separating epithelium and Bowman's membrane, the separating pressure is flexibly adjusted when separator 2 moves upward and downward on cornea, thus settling the problem of damage to corneal stroma due to the squeezing and piling up effect by separator 2 during moving upward and downward on cornea, as well as the rigid separating pressure on corneal stroma by separator 2. No matter separating piece 3 is made of metal or non-metal, its sharpness should not be too great so as to avoid cutting the stroma layer and assure the precise separation of corneal epithelium by mechanical force.

Moreover, no matter operation design is beeline or rotating, the advancing speed of separator 2 is not greater than 1.5 mm/sec, the pitching-in angle of separating piece 3 against cornea is not greater than 35°, the swing frequency is not less than 12000 rpm, and the swing amplitude is not less than 0.5 mm.

From above, the device according to the present invention has the following features: 1) automatic separation of epithelium using mechanical force; 2) the separator can be repeatedly used after sterilization, no consumption goods; 3) no alcohol immersion; 4) no damage to stroma layer; 5) simple device and convenient operation.

The above exemplary embodiment is provided only for explanation of the present invention as claimed. Those skilled in the related technical fields can make variations and changes not departing from the scope of the present invention as claimed. All equivalent technical schemes, therefore, should be within the scope of the present invention and be defined by the claims.

## Claims

1. An automatic corneal epithelium separation device including:
a dual-motor assembly (1) comprising two motors (7, 8) and a connecting key (13) fixed below the assembly;
a separator (2), with an inner-round via-hole (18) connecting up and down positioned inside, a separating piece (3) positioned on its one side and a check arm (16) extending out on its upper surface; and
a suction ring assembly comprising a suction ring (5), on the upper plane of which a suction ring stud (12) is fixed, on the ring face of which a flat key slot (14) is positioned, and a connecting tube (4) is connected with the suction ring (5) integrally through a stop ring fixed at its one end;
**characterized in that** the advancing speed of the separator (2) is not greater than 1.5 mm/sec and the pitching-in angle between the separating piece (3) and the cornea is less than 35°.

2. The automatic corneal epithelium separation device according to claim 1, **characterized in that** the swing frequency of the separating piece (3) is higher than 12000 rpm.

3. The automatic corneal epithelium separation device according to claim 1, **characterized in that** the swing amplitude of the separating piece (3) is larger than 0.5mm.

4. The automatic corneal epithelium separation device according to claim 1, **characterized in that** the separator (2) is a dock-styled flexible separator, and the floating range of the separating piece (3) in the separator is less than 200 µm.

## Patentansprüche

1. Automatisches Hornhautepithel-Trenngerät, umfassend
eine Doppelmotor-Anordnung (1), umfassend zwei Motoren (7, 8) und einen Verbindungskeil (13), der unter der Anordnung befestigt ist, einen Separator (2), mit einem innen runden Durchgangsloch (18), das oben und unten verbindet und in demselben ausgebildet ist, mit einem Trennelement (3), das auf einer seiner Seiten positioniert ist, sowie mit einem Sperrarm (16), der auf seiner oberen Fläche nach außen vorsteht, und
eine Saugringanordnung, die einen Saugring (5) umfasst, auf dessen oberer Ebene ein Saugringbolzen (12) befestigt ist, auf dessen Ringfläche ein flacher Keilschlitz (14) angeordnet ist, und wobei ein Verbindungsrohr (4) integral mit dem Saugring (5) durch einen an dessen einem Ende befestigten Anschlagring verbunden ist,
**dadurch gekennzeichnet, dass** die Vorschubgeschwindigkeit des Separators (2) nicht größer als 1,5 mm/s ist und der Neigungswinkel zwischen dem Trennelement (3) und der Hornhaut weniger als 35° beträgt.

2. Automatisches Hornhautepithel-Trenngerät nach Anspruch 1, **dadurch gekennzeichnet, dass** die Schwingfrequenz des Trennelements (3) größer als 12000 U/min ist.

3. Automatisches Hornhautepithel-Trenngerät nach Anspruch 1, **dadurch gekennzeichnet, dass** die Schwingamplitude des Trennelements (3) größer als 0,5 mm ist.

4. Automatisches Hornhautepithel-Trenngerät nach Anspruch 1, **dadurch gekennzeichnet, dass** der Separator (2) ein dock-förmiger, flexibler Separator ist und der Gleitbereich des Trennelements (3) in dem Separator weniger als 200 µm beträgt.

## Revendications

1. Appareil automatique de séparation de l'épithélium cornéen, incluant :
un ensemble à deux moteurs (1) comprenant deux moteurs (7, 8) et une clavette de liaison (13) fixée en dessous de l'ensemble ;
un séparateur (2), muni d'un trou d'interconnexion rond interne (18) placé à l'intérieur, assurant la liaison vers le haut et vers le bas, une pièce de séparation (3) placée sur un côté, et un bras d'arrêt (16) qui s'étend vers l'extérieur sur sa surface supérieure ; et
un ensemble d'anneau d'aspiration comprenant un anneau d'aspiration (5), sur le plan supérieur duquel est fixé un doigt d'anneau d'aspiration (12), sur la surface d'anneau duquel est agencée une fente de clavette plate (14), et un tube de liaison raccordé d'une seule pièce à l'anneau d'aspiration (5), par l'intermédiaire d'une bague de retenue fixée à l'une de ses extrémités ;
**caractérisé en ce que** la vitesse d'avance du séparateur (2) n'est pas supérieure à 1,5 mm/s, et l'angle d'attaque entre la pièce de séparation (3) et la cornée est inférieur à 35°.

2. Appareil automatique de séparation de l'épithélium cornéen selon la revendication 1, **caractérisé en ce que** la fréquence d'oscillation de la pièce de séparation (3) est supérieure à 12.000 t/mn.

3. Appareil automatique de séparation de l'épithélium cornéen selon la revendication 1, **caractérisé en ce que** l'amplitude d'oscillation de la pièce de séparation (3) est supérieure à 0,5 mm.

4. Appareil automatique de séparation de l'épithélium cornéen selon la revendication 1, **caractérisé en ce que** le séparateur (2) est un séparateur souple en forme de socle d'accueil, et la plage de flottaison de la pièce de séparation (3) dans le séparateur est inférieure à 200 µm.
